(19) Europäisches Patentamt
European Patent Office
Office européen des brevets

(11) **EP 4 442 326 A1**

(12) **EUROPEAN PATENT APPLICATION**
published in accordance with Art. 153(4) EPC

(43) Date of publication:
**09.10.2024 Bulletin 2024/41**

(21) Application number: **22901448.5**

(22) Date of filing: **02.12.2022**

(51) International Patent Classification (IPC):
***A61Q 5/02*** (2006.01) ***A61Q 5/12*** (2006.01)
***A61K 8/34*** (2006.01) ***A61K 8/36*** (2006.01)
***A61K 8/39*** (2006.01) ***A61K 8/46*** (2006.01)
***A61K 8/86*** (2006.01)

(52) Cooperative Patent Classification (CPC):
**A61K 8/34; A61K 8/36; A61K 8/39; A61K 8/44; A61K 8/46; A61K 8/86; A61Q 5/02; A61Q 5/12**

(86) International application number:
**PCT/JP2022/044607**

(87) International publication number:
**WO 2023/101018 (08.06.2023 Gazette 2023/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.12.2021 JP 2021197232**

(71) Applicant: **Kao Corporation**
**Tokyo 103-8210 (JP)**

(72) Inventors:
- **IMAI, Megumi**
  **Tokyo 131-8501 (JP)**
- **EZURE, Mikako**
  **Tokyo 131-8501 (JP)**

(74) Representative: **Hoffmann Eitle**
**Patent- und Rechtsanwälte PartmbB**
**Arabellastraße 30**
**81925 München (DE)**

(54) **HAIR CLEANSING COMPOSITION**

(57) A hair cleansing composition comprising the following components (a), (b), and (c): (a) one or more compounds selected from the group consisting of a polyalkylene glycol ether and an alkylene glycol at 0.1% by mass or more and 18% by mass or less; (b) one or more selected from the group consisting of a carboxylic acid-type anionic surfactant, a sulfonic acid-type anionic surfactant, an ether sulfate-type anionic surfactant, and an amphoteric surfactant at 5% by mass or more; and (c) an organic acid or a salt thereof.

Fig. 1

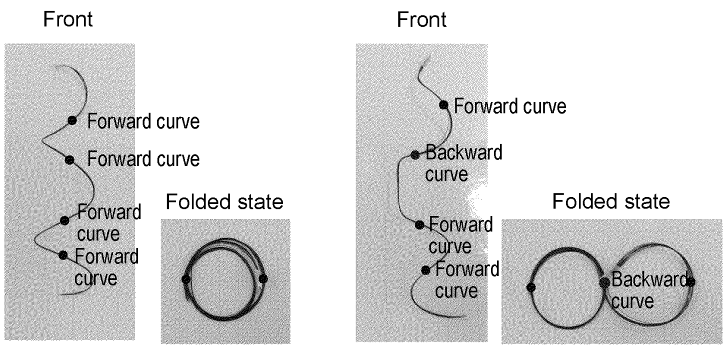


$\frac{4}{4} \times 100 = 100\ \%$ $\frac{3}{4} \times 100 = 75\ \%$

EP 4 442 326 A1

## Description

Field of the Invention

The present invention relates to a hair cleansing composition.

Background of the Invention

Conventionally, various hair cosmetics have been proposed for the purpose of finishing waves or curls of hair in a desirable state and of keeping these beautiful (for example, Patent Literatures 1 to 4).

Citation List

Patent Literatures

(Patent Literature 1) JP-A-1992-230614
(Patent Literature 2) JP-A-2015-105271
(Patent Literature 3) JP-A-2012-131784
(Patent Literature 4) JP-A-2020-186222

Summary of the Invention

The present invention provides a hair cleansing composition containing the following components (a), (b), and (c):

(a) one or more compounds selected from the group consisting of a polyalkylene glycol ether and an alkylene glycol at 0.1% by mass or more and 18% by mass or less;
(b) one or more selected from the group consisting of a carboxylic acid-type anionic surfactant, a sulfonic acid-type anionic surfactant, an ether sulfate-type anionic surfactant, and an amphoteric surfactant at 5% by mass or more; and
(c) an organic acid or a salt thereof.

Brief Description of Drawing

Figure 1 illustrates a method for calculating a spiral rate.

Detailed Description of the Invention

The hair cosmetics disclosed in Patent Literatures 1 to 3 impart elasticity to hair or gather tresses by adhesion between the hair surfaces, and are not intended at all to control the structure of the tresses to make waves or curls look beautiful.

Patent Literature 4 discloses that calcium ions in water are accumulated in the hair during daily hair washing, causing a problem such as the direction of the curl partially curving backward to form a random shape which impairs the beauty thereof, but the treatment of the hair with three specific hair treatment agents in a predetermined order allows the calcium ions accumulated in the hair to flow out and reforms the inside of the hair to form a beautiful curl shape having uniformly consistent phases. However, the hair treatment disclosed in Patent Literature 4 employs three agents and is a treatment involving standing time and heating, and thus it is complicated and time-consuming for consumers.

Therefore, the present invention relates to a hair cleansing composition which can control the structure of tresses to form a curl-consistent, three-dimensional, manageable, and beautiful waves or curls only by daily hair care action of hair washing.

The present inventor found that a beautiful waves or curls can be formed with high reproducibility by allowing tresses in a wet state to have a spiral structure. Furthermore, the present inventor found that washing hair having waves or curls by using a hair cleansing agent containing a specific compound can make the wet tresses after hair washing likely to form a spiral shape and can realize a curl-consistent, three-dimensional, manageable, and beautiful waves or curls, and completed the present invention.

The hair cleansing composition of the present invention can make wet tresses after hair washing likely to form a spiral shape and can form a curl-consistent, three-dimensional, manageable, and beautiful waves or curls.

[Component (a): one or more compounds selected from the group consisting of polyalkylene glycol ether and alkylene glycol]

The component (a) is one or more compounds selected from the group consisting of a polyalkylene glycol ether and an alkylene glycol. Examples of the polyalkylene glycol ether include a compound of the following formula (1) :

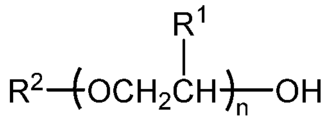

(1)

wherein $R^1$ represents a hydrogen atom or a methyl group, $R^2$ represents an alkyl group having from 1 to 5 carbon atoms, and n represents a number of from 2 to 6.

In the formula (1), $R^1$ is preferably a hydrogen atom, $R^2$ is preferably an alkyl group having from 2 to 4 carbon atoms, and n is preferably a number of from 2 to 3.

Examples of the compound of the formula (1) include diethylene glycol monomethyl ether (methyl carbitol), diethylene glycol monoethyl ether (ethyl carbitol), diethylene glycol monopropyl ether (propyl carbitol), diethylene glycol monobutyl ether (butyl carbitol), triethylene glycol monomethyl ether, and triethylene glycol monoethyl ether, and from the viewpoint of making wet tresses after hair washing likely to form a spiral shape, diethylene glycol monoethyl ether (ethyl carbitol), diethylene glycol monobutyl ether (butyl carbitol), and triethylene glycol monomethyl ether are preferable.

Examples of the alkylene glycol include ethylene glycol, 1,3-propanediol, 1,3-butanediol, and 3-methyl-1,3-butanediol (isoprene glycol), and from the viewpoint of making wet tresses after hair washing likely to form a spiral shape, 3-methyl-1,3-butanediol (isoprene glycol) is preferable.

From the viewpoint of making wet tresses after hair washing likely to form a spiral shape and of improving the manageability and uniformity of a curl, among the above, one or more selected from the group consisting of diethylene glycol monoethyl ether (ethyl carbitol), diethylene glycol monobutyl ether (butyl carbitol), triethylene glycol monomethyl ether, and 3-methyl-1,3-butanediol (isoprene glycol) are preferable, one or more selected from the group consisting of diethylene glycol monoethyl ether (ethyl carbitol), diethylene glycol monobutyl ether (butyl carbitol), and triethylene glycol monomethyl ether are more preferable, one or more selected from the group consisting of diethylene glycol monoethyl ether (ethyl carbitol) and diethylene glycol monobutyl ether (butyl carbitol) are further more preferable, and diethylene glycol monoethyl ether (ethyl carbitol) is even more preferably included.

Such components (a) can be used singly or in combination of two or more, and from the viewpoint of making wet tresses after hair washing likely to form a spiral shape and of improving the manageability and uniformity of a curl, the content thereof in the hair cleansing composition is 0.1% by mass or more, preferably 0.5% by mass or more, more preferably 1.0% by mass or more, further more preferably 2.0% by mass or more, and even more preferably 3.0% by mass or more, and in addition, from the viewpoint of making wet tresses after hair washing likely to form a spiral shape and from the viewpoint of keeping a suitable viscosity as a cleansing agent, the content is 18% by mass or less, preferably 15% by mass or less, more preferably 12% by mass or less, and further more preferably 10% by mass or less. The specific range of the content of the component (a) in the hair cleansing composition is from 0.1 to 18% by mass, preferably from 0.5 to 15% by mass, more preferably from 1.0 to 15% by mass, further more preferably from 2.0 to 12% by mass, and even more preferably from 3.0 to 10% by mass.

[Component (b): one or more selected from the group consisting of carboxylic acid-type anionic surfactant, sulfonic acid-type anionic surfactant, ether sulfate-type anionic surfactant, and amphoteric surfactant]

Examples of the carboxylic acid-type anionic surfactant include an N-acylamino acid salt, an N-acyl-N-alkylamino acid salt, an amide type N-acylamino acid salt, an alkyl ether carboxylate, a fatty acid salt, and a salt of an alkyl succinate or an alkenyl succinate, examples of the sulfonic acid-type anionic surfactant include a sulfosuccinate-type, an isethionate-type, a taurate-type, an alkylbenzenesulfonate-type, an $\alpha$-olefin sulfonate-type, and an alkanesulfonic acid-type, and examples of the ether sulfate-type anionic surfactant include a polyoxyethylene alkyl ether sulfate and an alkyl ether sulfate. Examples of the amphoteric surfactant include imidazoline, carbobetaine, amidobetaine, sulfobetaine, hydroxysulfobetaine, and amidosulfobetaine. Such components (b) can be used singly or in combination of two or more, and it is more preferable to contain one or more anionic surfactants selected from the group consisting of a carboxylic acid-type anionic surfactant, a sulfonic acid-type anionic surfactant, and an ether sulfate-type anionic surfactant and one or more amphoteric surfactants.

From the viewpoint of making wet tresses after hair washing likely to form a spiral shape and of improving the manageability and uniformity of a curl, the anionic surfactant is preferably one or more selected from the group consisting of an N-acylamino acid salt, an alkyl ether carboxylate, an $\alpha$-olefin sulfonate, a polyoxyethylene alkyl ether sulfate, and

an alkyl ether sulfate, more preferably one or more selected from the group consisting of an alkyl ether carboxylate, an α-olefin sulfonate, a polyoxyethylene alkyl ether sulfate, and an alkyl ether sulfate, and further more preferably one or more selected from the group consisting of an α-olefin sulfonate and a polyoxyethylene alkyl ether sulfate, and a polyoxyethylene alkyl ether sulfate is even more preferably included.

In the N-acylamino acid salt, from the above viewpoint, the acyl group of the N-acylamino acid salt is preferably a fatty acid residue having from 8 to 14 carbon atoms, and the amino acid moiety of the N-acylamino acid salt is preferably a neutral amino acid selected from the group consisting of glycine and alanine or an acidic amino acid selected from the group consisting of glutamic acid and aspartic acid, more preferably an acidic amino acid, and further more preferably glutamic acid. Specifically, the N-acylamino acid salt is preferably one or more selected from the group consisting of an N-lauroyl glutamate, an N-myristoyl glutamate, an N-cocoyl glutamate, and an N-lauroyl aspartate, more preferably one or more selected from the group consisting of an N-lauroyl glutamate, an N-myristoyl glutamate, and an N-cocoyl glutamate, and an N-cocoyl glutamate is further more preferably included.

From the above viewpoint, the alkyl ether carboxylate is preferably an alkyl ether carboxylate having an alkyl group having from 8 to 14 carbon atoms, and more preferably an alkyl ether carboxylate having an alkyl group having from 8 to 14 carbon atoms and having an average number of moles of an ethylene oxide group added of from 1 to 5.

From the above viewpoint, the α-olefin sulfonate is preferably an α-olefin sulfonate having an α-olefin having an alkyl group having from 10 to 18 carbon atoms, more preferably one having from 12 to 16 carbon atoms, and further more preferably a tetradecene sulfonate having 14 carbon atoms.

From the above viewpoint, the polyoxyethylene alkyl ether sulfate and the alkyl ether sulfate are preferably a polyoxyethylene alkyl ether sulfate and an alkyl ether sulfate having an alkyl group having from 8 to 14 carbon atoms, and particularly preferably a polyoxyethylene alkyl ether sulfate having an alkyl group having from 8 to 14 carbon atoms and having an average number of moles of an ethylene oxide group added of from 1 to 5.

Examples of a counter ion of an anionic group of such an anionic surfactant include an alkali metal ion such as a sodium ion or a potassium ion; an alkaline earth metal ion such as a calcium ion or a magnesium ion; an ammonium ion; and an alkanolammonium having from 1 to 3 alkanol groups each having 2 or 3 carbon atoms (for example, monoethanolammonium, diethanolammonium, triethanolammonium, or triisopropanolammonium).

From the viewpoint of making wet tresses after hair washing likely to form a spiral shape and of improving the manageability and uniformity of a curl, the amphoteric surfactant is preferably one or more selected from the group consisting of carbobetaine and sulfobetaine; more specifically, the amphoteric surfactant is preferably one or more selected from the group consisting of a fatty acid amidoalkyl betaine and an alkyl hydroxysulfobetaine, more preferably one or more selected from the group consisting of a fatty acid amidopropyl betaine having a fatty acid group having from 8 to 14 carbon atoms and an alkyl hydroxysulfobetaine having an alkyl group having from 8 to 14 carbon atoms, and further more preferably one or more selected from the group consisting of lauramidopropyl betaine and lauryl hydroxysultaine.

From the viewpoint of making wet tresses after hair washing likely to form a spiral shape and of improving the manageability and uniformity of a curl, the content of the component (b) in the hair cleansing composition is 5% by mass or more, preferably 8% by mass or more, and more preferably 12% by mass or more, and is preferably 30% by mass or less, more preferably 25% by mass or less, and further more preferably 20% by mass or less.

[Component (c): organic acid or salt thereof]

From the viewpoint of making wet tresses after hair washing likely to form a spiral shape and of improving the manageability and uniformity of a curl, the organic acid of the component (c) is preferably an organic acid having 8 or less carbon atoms, more preferably a dicarboxylic acid, a tricarboxylic acid, a hydroxycarboxylic acid, or a carboxylic acid having an aldehyde group, and further more preferably a hydroxycarboxylic acid, each having 8 or less carbon atoms. Specifically, examples of the dicarboxylic acid include malonic acid, succinic acid, glutaric acid, adipic acid, maleic acid, fumaric acid, phthalic acid, oxalic acid, malic acid, and tartaric acid, examples of the tricarboxylic acid include citric acid, examples of the hydroxycarboxylic acid include glycolic acid, lactic acid, hydroxyacrylic acid, oxybutyric acid, glyceric acid, malic acid, tartaric acid, and citric acid, and examples of the carboxylic acid having an aldehyde group include glyoxylic acid. Among these, the organic acid is preferably one or more hydroxycarboxylic acids selected from the group consisting of glycolic acid, lactic acid, malic acid, tartaric acid, and citric acid, more preferably one or more hydroxycarboxylic acids selected from the group consisting of lactic acid, malic acid, and tartaric acid, and further more preferably one or more hydroxycarboxylic acids selected from the group consisting of lactic acid and malic acid. In addition, examples of a salt of such an organic acid include a salt with an alkali metal, an alkaline earth metal, ammonia, or an organic compound. These organic acids or salts thereof can be used singly or in combinations of two or more.

From the viewpoint of improving the persistence of the component (a) on the hair and of making wet tresses after hair washing likely to form a spiral shape, the content of the component (c) in the hair cleansing composition is preferably 0.1% by mass or more, more preferably 0.2% by mass or more, and further more preferably 0.5% by mass

or more, and from the viewpoint of suppressing friction during rinsing and of making wet tresses after hair washing likely to form a spiral shape, the content is preferably 5% by mass or less, more preferably 2% by mass or less, and further more preferably 1% by mass or less.

From the viewpoint of making wet tresses after hair washing likely to form a spiral shape and of improving the manageability and uniformity of a curl, the mass ratio of the component (a) to the component (c), (a)/(c), in the hair cleansing composition of the present invention is preferably 0.5 or more, more preferably 1.0 or more, further more preferably 3 or more, and even more preferably 5 or more, and is preferably 100 or less, more preferably 50 or less, and further more preferably 30 or less, and even more preferably 25 or less.

[Component (d): nonionic surfactant]

The hair cleansing composition of the present invention preferably further contains a nonionic surfactant as component (d). Examples of the nonionic surfactant include an alkyl glucoside, a sorbitan fatty acid ester, a glycerin fatty acid ester, a polyglycerin fatty acid ester, a propylene glycol fatty acid ester, a polyethylene glycol fatty acid ester, a sucrose fatty acid ester, a polyoxyethylene fatty acid ester, a polyoxyethylene sorbitan fatty acid ester, a polyoxyethylene alkyl ether, a polyoxypropylene alkyl ether, an alkyl glyceryl ether, a polyoxyethylene sorbitol fatty acid ester, a polyoxyethylene glycerin fatty acid ester, a polyoxyethylene propylene glycol fatty acid ester, polyoxyethylene castor oil, polyoxyethylene hydrogenated castor oil, a polyoxyethylene hydrogenated castor oil fatty acid ester, polyoxyethylene phytostanol ether, polyoxyethylene phytosterol ether, polyoxyethylene cholestanol ether, polyoxyethylene cholesteryl ether, a polyoxyalkylene modified organopolysiloxane, and a polyoxyalkylene/alkyl co-modified organopolysiloxane. From the viewpoint of making wet tresses after hair washing likely to form a spiral shape, among these, the nonionic surfactant is preferably one or more selected from the group consisting of an alkyl glucoside, a polyoxyethylene alkyl ether, a polyoxypropylene alkyl ether, and an alkyl glyceryl ether, more preferably one or more selected from the group consisting of an alkyl glucoside, a polyoxypropylene alkyl ether, and an alkyl glyceryl ether, and further more preferably an alkyl glucoside. Such components (d) can be used singly or in combination of two or more.

From the above viewpoint, the alkyl glucoside is an alkyl glucoside having an alkyl group having preferably from 8 to 22 carbon atoms, more preferably from 8 to 18 carbon atoms, and further more preferably from 8 to 12 carbon atoms. Specific examples thereof include "MYDOL 10" (decyl glucoside) and "AG-124" (lauryl glucoside) manufactured by Kao Corporation.

From the above viewpoint, the polyoxyethylene alkyl ether is a polyoxyethylene alkyl ether having an alkyl group having preferably from 8 to 22 carbon atoms, more preferably from 8 to 18 carbon atoms, and further more preferably from 8 to 12 carbon atoms. Specific examples thereof include "EMULGEN 103" (laureth-3), "EMULGEN 116" (laureth-16), and "EMULGEN 306P" (steareth-6) manufactured by Kao Corporation.

From the above viewpoint, the polyoxypropylene alkyl ether is a polyoxypropylene alkyl ether having an alkyl group having preferably from 8 to 22 carbon atoms, more preferably from 8 to 18 carbon atoms, and further more preferably from 8 to 12 carbon atoms. Specific examples thereof include "KAO SOFCARE GP-1" (PPG-3 caprylyl ether) manufactured by Kao Corporation.

From the above viewpoint, the alkyl glyceryl ether is an alkyl glyceryl ether having an alkyl group having preferably from 8 to 22 carbon atoms, more preferably from 8 to 18 carbon atoms, and further more preferably from 8 to 12 carbon atoms, and specific examples thereof include "PENETOL GE-ID" (isodecyl glyceryl ether) manufactured by Kao Corporation.

From the viewpoint of improving the persistence of the component (a) on the hair and of making wet tresses after hair washing likely to form a spiral shape, the content of the component (d) in the hair cleansing composition is preferably 0.1% by mass or more, more preferably 0.5% by mass or more, and further more preferably 1% by mass or more, and from the viewpoint of improving the natural use impression and the storage stability and of making wet tresses after hair washing likely to form a spiral shape, the content is preferably 10% by mass or less, more preferably 8% by mass or less, and further more preferably 5% by mass or less.

[Component (e): cationic polymer]

The hair cleansing composition of the present invention preferably further contains a cationic polymer as component (e) in order to impart a smooth feel during hair washing and rinsing. Specific examples of the cationic polymer include cationized cellulose, cationic starch, cationized guar gum, a diallyl quaternary ammonium salt/acrylamide copolymer, a vinylimidazolium trichloride/vinylpyrrolidone copolymer, a hydroxyethylcellulose/dimethyldiallylammonium chloride copolymer, a vinylpyrrolidone/quaternized dimethylaminoethyl methacrylate copolymer, a polyvinylpyrrolidone/alkylaminoacrylate copolymer, a polyvinylpyrrolidone/alkylaminoacrylate/vinylcaprolactam copolymer, a vinylpyrrolidone/methacrylamidopropyl trimethylammonium chloride copolymer, an alkylacrylamide/acrylate/alkylaminoalkylacrylamide/polyet hylene glycol methacrylate copolymer, an adipic acid/dimethylaminohydroxypropylethylenetriamine copolymer (Cartaretin, Sandoz, USA), and cationic polymers disclosed in JP-A-H11-71435, JP-A-S53-139734, and JP-AS60-36407.

In addition, examples of a commercially available product thereof which can be used include Merquat 550 (Lubrizol, copolymer of acrylamide and diallyldimethylammonium salt; INCI name: polyquaternium-7), Luviquat FC370 (BASF, copolymer of 1-vinyl-2-pyrrolidone and 1-vinyl-3-methylimidazolium salt; INCI name: polyquaternium-16), Gafquat 755N (Ashland Inc., copolymer of 1-vinyl-2-pyrrolidone and dimethylaminoethyl methacrylate; INCI name: polyquaternium-11), Ucare Polymer JR series and Ucare Polymer LR series (Amerchol Corporation, salt of reaction product between trimethylammonium-substituted epoxide and hydroxyethylcellulose; INCI name: polyquaternium-10), POIZ C-60H, POIZ C-80M, and POIZ C-150L (Kao Corporation, salt of reaction product between trimethylammonium-substituted epoxide and hydroxyethylcellulose; INCI name: polyquaternium-10), CATICELLO M-80 and CATICELLO (Kao Corporation, cationized hydroxyethylcellulose; INCI name: polyquaternium-10), SOFCARE KG-301W and SOFCARE KG-101W-E (Kao Corporation, N,N-dimethylaminoethyl methacrylate diethylsulfate/N,N-dimethyl acrylamide/polyethylene glycol dimethacrylate copolymer; INCI name: polyquaternium-52), and Jaguar series (Solvay, guar hydroxypropyltriammonium chloride).

From the viewpoint of making wet tresses after hair washing likely to form a spiral shape and of improving the manageability and uniformity of a curl, among the above, a cationized polysaccharide such as cationized cellulose, cationized starch, cationized guar gum, cationized tara gum, cationized fenugreek gum, or cationized locust bean gum is preferable, one or more selected from the group consisting of cationized cellulose and cationized guar gum is more preferable, and cationized cellulose is further more preferable. The degree of cationic substitution of cationized cellulose is preferably from 0.01 to 1, and the molecular weight thereof is preferably from 100 000 to 8 000 000.

Such components (e) can be used singly or in combination of two or more, and from the viewpoint of making wet tresses after hair washing likely to form a spiral shape and improving manageability and uniformity of a curl, the content of the component (e) in the hair cleansing composition is preferably 0.01% by mass or more, more preferably 0.1% by mass or more, and further more preferably 0.2% by mass or more, and is preferably 5% by mass or less, more preferably 3% by mass or less, and further more preferably 1% by mass or less.

[Other optional components]

Other component commonly used in a hair cleansing agent can be further blended into the hair cleansing composition of the present invention, depending on the purpose. Examples thereof include an ester oil such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate, or tridecyl isononanoate; a wax such as beeswax, whale wax, lanolin, or carnauba wax; a solvent such as benzyl alcohol, phenoxyethanol, 2-benzyloxyethanol, or glycerin; a silicone such as dimethylpolysiloxane or amino-modified silicone; an antidandruff agent such as zinc pyrithione or benzalkonium chloride; a vitamin; a microbicide; an antiinflammatory agent; an antiseptic; a chelating agent; a moisturizing agent such as panthenol; a colorant such as a dye or a pigment; an extract such as a polar solvent extract of eucalyptus, a protein obtained from a shell or a pearl having a nacreous layer or a hydrolysate thereof, a protein obtained from silk or a hydrolysate thereof, a protein-containing extract obtained from a seed of a leguminous plant, an Asian ginseng extract, a rice germ extract, a fucus extract, a camellia extract, an aloe extract, a shellplant leaf extract, or a chlorella extract; a pearl powder such as titanated mica; a fragrance; a coloring; an ultraviolet absorber; an antioxidant; and other components disclosed in

ENCYCLOPEDIA OF SHAMPOO INGREDIENTS (MICELLE PRESS).

The hair cleansing composition of the present invention uses water as a medium, and a lower alcohol such as ethanol or 1,3-butylene glycol can also be further used in combination as a medium other than water.

From the viewpoint of imparting a smooth feel during hair washing and rinsing or improving the setting property of the hair, the hair cleansing composition of the present invention can further contain an anionic or amphoteric film-forming polymer. However, the anionic or amphoteric film-forming polymer may reduce the formation of a spiral shape without a backward curve or reduce the reproducibility of a curl, and thus the content thereof in the hair cleansing composition is preferably 5% by mass or less, more preferably 1% by mass or less, further more preferably 0.5% by mass or less, and even more preferably 0.1% by mass or less, and the hair cleansing composition is even more preferably substantially free of an anionic or amphoteric film-forming polymer.

In the present invention, "substantially free" means that the content in the composition is less than 0.01% by mass and preferably 0% by mass.

In addition, in the present invention, the film-forming polymer means a polymer capable of forming a film on a keratin substance when it is dried.

Hereinafter, the film-forming polymer will be exemplified.

<Anionic film-forming polymer>

- Anionic film-forming polymer including any of crotonic acid, maleic acid, maleic acid monoester, and itaconic acid as constitutional unit:
  The anionic film-forming polymer is not particularly limited as long as it includes any of crotonic acid, maleic acid, maleic acid monoester, and itaconic acid as a constitutional unit, and examples thereof include one obtained by copolymerization with one or more of a vinyl ether, a vinyl ester, and a vinyl alcohol. Specific examples thereof include a methyl vinyl ether/alkyl maleate copolymer (for example, Gantrez ES-225, Gantrez ES-425, and Gantrez SP-215 manufactured by ISP), a vinyl acetate/crotonic acid copolymer (for example, Resin 28-1310 manufactured by National Starch), a vinyl acetate/crotonic acid/vinyl neodecanoate copolymer (for example, Resin 28-2930 manufactured by National Starch), a vinyl acetate/crotonic acid/vinyl propionate copolymer (for example, Luviset CAP manufactured by BASF), and a vinyl alcohol/itaconic acid copolymer (for example, KM-118 manufactured by Kuraray Co., Ltd.).
- Anionic film-forming polymer including (meth)acrylic acid as constitutional unit:
  Examples thereof include a vinyl polymer including (meth)acrylic acid as a constitutional unit. Specific examples thereof include an acrylic acid/ethyl acrylate/N-t-butylacrylamide copolymer (for example, Ultrahold 8 and Ultrahold Strong manufactured by BASF), an octylacrylamide/acrylic acid copolymer (for example, Amphomer V-42 manufactured by National Starch), an acrylate/methacrylate/acrylic acid/methacrylic acid copolymer (for example, Amerhold DR25 manufactured by Union Carbide Corporation), an acrylates/diacetoneacrylamide copolymer (for example, Plascize L9540B (product obtained by neutralizing the polymer with 2-amino-2-methyl-1-propyl alcohol) manufactured by GOO Chemical Co., Ltd.), and an acrylates/C1-18 alkyl acrylates/C1-8 alkyl acrylamide copolymer (for example, Plascize L9909B (product obtained by neutralizing the polymer with 2-amino-2-methyl-1-propyl alcohol) manufactured by Goo Chemical Industry Co., Ltd.).
- Natural anionic film-forming polymer having either a carboxy group or a sulfuric acid group:
  The natural anionic film-forming polymer is not particularly limited as long as it is a natural polysaccharide having either a carboxyl group or a sulfuric acid group, and examples thereof include carrageenan (for example, Soageena LX22 and Soageena ML210 manufactured by Mitsubishi Rayon Co., Ltd.), and xanthan gum (for example, ECHO GUM T manufactured by Dainippon Pharmaceutical Co., Ltd.).
- Polyester having either carboxy group or sulfonic acid group:
  The polyester is not particularly limited, and examples thereof include a water-dispersible polyester (for example, AQ38S and AQ55S manufactured by the Eastman Kodak Company).

<Amphoteric film-forming polymer>

The amphoteric film-forming polymer is not particularly limited, and examples thereof include a vinyl polymer. Here, the amphoteric polymer contains both a copolymer including one or more anionic monomers and one or more cationic monomers as constitutional units, and a copolymer including as constitutional units one or more so-called betaine monomers having both an anionic moiety and a cationic moiety in the monomer structure.

Specific examples thereof include a dimethyldiallylammonium/acrylamide/acrylic acid copolymer (for example, Merquat 3331 manufactured by Calgon), an N-methacryloyloxyethyl-N,N-dimethylammonium-$\alpha$-N-methylcarboxybetaine/methacrylic acid alkyl ester copolymer (for example, Yukaformer M-75 and Yukaformer SM manufactured by Mitsubishi Chemical Corporation); and an octylacrylamide/acrylate/butylaminoethyl methacrylate copolymer (for example, Amphomer 28-4910 manufactured by National Starch).

The hair cleansing composition of the present invention can further contain a reductive compound. However, among reductive compounds, a compound having a thiol group or an aldehyde group, a sulfite, and a sulfinic acid may accelerate hair damage and additionally reduce the formation of a spiral shape without a backward curve or the reproducibility of a curl, and thus the hair cleansing composition is preferably substantially free of these. In addition, a reductive compound having an ascorbyl group may also cause the above problem depending on the content, and thus the content thereof in the hair cleansing composition is preferably less than 1% by mass, more preferably less than 0.5% by mass, further more preferably less than 0.1% by mass, and even more preferably less than 0.05% by mass.

Specific examples of a reductive compound having a thiol group include thioglycolic acid, thiolactic acid, cysteine, cysteamine, homocysteine, glutathione, thioglycerol, thiomalic acid, 2-mercaptopropionic acid, 3-mercaptopropionic acid, thiodiglycol, 2-mercaptoethanol, dithiothreitol, N-acetylcysteine, and an ester and an amide of thioglycolic acid or thiolactic acid.

Specific examples of a reductive compound having an aldehyde group include formaldehyde, 1,3,5-trioxane, paraformaldehyde, and formalin. Similarly, methylene glycol is a compound present in equilibrium in an aqueous formaldehyde solution and is substantially synonymous with formalin and thus shall be included in the definition thereof.

Examples of the sulfite include ammonium sulfite, ammonium hydrogen sulfite, sodium sulfite, sodium hydrogen sulfite, potassium sulfite, and potassium hydrogen sulfite.

Examples of the sulfinic acid include a sulfinate and a benzenesulfinate.

Examples of the reductive compound having an ascorbyl group include ascorbic acid, erythorbic acid, and salts or esters thereof. Specific examples thereof include ascorbic acid, sodium ascorbate, potassium ascorbate, calcium ascorbate, ammonium ascorbate, monoethanolamine ascorbate, diethanolamine ascorbate, erythorbic acid, sodium erythorbate, disodium ascorbyl sulfate, magnesium ascorbyl phosphate, ascorbyl palmitate, ascorbyl stearate, ascorbyl dipalmitate, ascorbyl tetra-2-hexyldecanoate, ascorbyl myristate, ascorbyl laurate, ascorbyl acetate, ascorbyl propionate, ascorbyl tartrate, ascorbyl citrate, ascorbyl succinate, ascorbyl benzoate, potassium (ascorbyl/tocopheryl) phosphate, ethyl ascorbic acid, allantoin ascorbate, chitosan ascorbate, methylsilanol ascorbate, tetradecylhexyl ascorbate, aminopropyl ascorbyl phosphate, ascorbic acid polypeptide, ascorbyl glucoside, and ascorbyl methylsilanol pectinate.

[pH]

From the viewpoint of the storage stability of the formulation, the pH of the hair cleansing composition of the present invention is preferably 3 or more, more preferably 3.5 or more, further more preferably 4 or more, and even more preferably 5 or more, and from the viewpoint of reducing the damage to the hair or the scalp, the pH is preferably 8 or less, more preferably 7.5 or less, further more preferably 7 or less, and even more preferably 6 or less. In the present invention, the pH of the hair cleansing composition refers to the value at 25°C when the hair cleansing composition is diluted 20-fold by mass with water, and specifically means the value measured by the method described in Examples.

The dosage form of the hair cleansing composition of the present invention can be in any form such as a liquid, a milky lotion, a cream, or a foam.

[Method for using hair cleansing composition]

The hair cleansing composition of the present invention is applied to hair having waves or curls, preferably hair having waves or curls well moistened with water, let sit on the entire hair, lathered well, and then washed off when it is used. Subsequently, drying such as towel drying is optionally carried out, and a scrunch operation is preferably carried out on the hair in a wet state having a mass ratio of water to the hair (water/hair) of 0.05 or more and 1 or less. Here, in the present invention, the scrunch operation is preferably one or more operations selected from the group consisting of the following 1) to 3), and from the viewpoint of making wet tresses after hair washing likely to form a spiral shape and of improving the manageability and uniformity of a curl, it is preferable to carry out one or more operations selected from the group consisting of 1) and 3), and it is more preferable to carry out the operation of 3). During the scrunch operation, tresses may be placed on a towel spread over the palm of a hand.

1) The hair is grasped or massaged
2) The hair is twisted
3) The hair is placed in the palm of a hand, and then lifted up in such a way as to grasp up the hair and released

For the wet state of the hair when the scrunch operation is carried out, from the viewpoint of making wet tresses after hair washing likely to form a spiral shape and of improving the manageability and uniformity of a curl, the mass ratio of water to the hair (water/hair) is 0.05 or more and 1 or less, preferably 0.1 or more, more preferably 0.2 or more, further more preferably 0.3 or more, and even more preferably 0.5 or more, and is preferably 0.9 or less, and more preferably 0.8 or less.

From the viewpoint of making wet tresses after hair washing likely to form a spiral shape and of improving manageability and uniformity of a curl, the number of repetitions of the scrunch operation is preferably 1 time or more, more preferably 3 times or more, and further more preferably 5 times or more, for a handful of tresses, and from the viewpoint of labor reduction, the number is preferably 50 times or less, more preferably 30 times or less, and further more preferably 20 times or less.

The hair after scrunch treatment may optionally be subjected to a drying step or a styling step using a hair styling agent or the like.

By washing the hair by using the hair cleansing composition of the present invention as described above and then carrying out the scrunch operation, the wet tresses after hair washing can be made likely to form a spiral shape, and a curl-consistent, three-dimensional, manageable, and beautiful waves or curls can be formed.

In the present invention, the spiral structure of the wet tresses after hair washing can be evaluated by the spiral rate represented by the following equation.

Spiral rate (%) = (number of forward curve points/total number of curl points) × 100

wherein the "number of forward curve points" is the number of points where a certain curl curls in the same direction as half a turn before the curl when tresses are placed from the hair end in such a way as to fold the tresses and the direction of the curl is observed from the root side of the tresses.

Preferable aspects of the present invention with respect to the above embodiments will be further disclosed below.

<1> A hair cleansing composition comprising the following components (a), (b), and (c):

(a) one or more compounds selected from the group consisting of a polyalkylene glycol ether and an alkylene glycol at 0.1% by mass or more and 18% by mass or less; and
(b) one or more selected from the group consisting of a carboxylic acid-type anionic surfactant, a sulfonic acid-type anionic surfactant, an ether sulfate-type anionic surfactant, and an amphoteric surfactant at 5% by mass or more; and
(c) an organic acid or a salt thereof.

<2> The hair cleansing composition according to <1>, wherein the polyalkylene glycol ether in the component

(a) is preferably a compound of the following formula (1).

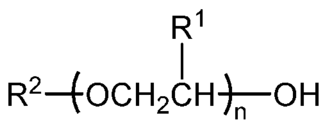

(1)

wherein $R^1$ represents a hydrogen atom or a methyl group, preferably a hydrogen atom, $R^2$ represents an alkyl group having from 1 to 5 carbon atoms, preferably from 2 to 4 carbon atoms, and n represents a number of from 2 to 6, preferably from 2 to 3.

<3> The hair cleansing composition according to <1> or <2>, wherein the polyalkylene glycol ether in the component (a) is preferably one or more selected from the group consisting of diethylene glycol monomethyl ether (methyl carbitol), diethylene glycol monoethyl ether (ethyl carbitol), diethylene glycol monopropyl ether (propyl carbitol), diethylene glycol monobutyl ether (butyl carbitol), triethylene glycol monomethyl ether, and triethylene glycol monoethyl ether, and more preferably one or more selected from the group consisting of diethylene glycol monoethyl ether (ethyl carbitol), diethylene glycol monobutyl ether (butyl carbitol), and triethylene glycol monomethyl ether.
<4> The hair cleansing composition according to any one of <1> to <3>, wherein the alkylene glycol in the component (a) is preferably one or more selected from the group consisting of ethylene glycol, 1,3-propanediol, 1,3-butanediol, and 3-methyl-1,3-butanediol (isoprene glycol), and more preferably 3-methyl-1,3-butanediol (isoprene glycol).
<5> The hair cleansing composition according to any one of <1> to <4>, wherein the component (a) comprises preferably one or more selected from the group consisting of diethylene glycol monoethyl ether (ethyl carbitol), diethylene glycol monobutyl ether (butyl carbitol), triethylene glycol monomethyl ether, and 3-methyl-1,3-butanediol (isoprene glycol), more preferably one or more selected from the group consisting of diethylene glycol monoethyl ether (ethyl carbitol), diethylene glycol monobutyl ether (butyl carbitol), and triethylene glycol monomethyl ether, further more preferably one or more selected from the group consisting of diethylene glycol monoethyl ether (ethyl carbitol) and diethylene glycol monobutyl ether (butyl carbitol), and further more preferably diethylene glycol monoethyl ether (ethyl carbitol).
<6> The hair cleansing composition according to any one of <1> to <5>, wherein a content of the component (a) is 0.1% by mass or more, preferably 0.5% by mass or more, more preferably 1.0% by mass or more, further more preferably 2.0% by mass or more, and further more preferably 3.0% by mass or more, and the content is 18% by mass or less, preferably 15% by mass or less, more preferably 12% by mass or less, and further more preferably 10% by mass or less.
<7> The hair cleansing composition according to any one of <1> to <6>, wherein the component (b) is preferably one or more selected from the group consisting of an N-acylamino acid salt, an N-acyl-N-alkylamino acid salt, an amide-type N-acylamino acid salt, an alkyl ether carboxylate, a fatty acid salt, a salt of an alkyl succinate or an alkenyl succinate, a sulfosuccinate-type anionic surfactant, an isethionate-type anionic surfactant, a taurate-type anionic surfactant, an alkylbenzenesulfonate-type anionic surfactant, an α-olefin sulfonate-type anionic surfactant, an alkanesulfonic acid-type anionic surfactant, a polyoxyalkyl ether sulfate, an alkyl ether sulfate, imidazoline, carbobetaine, amidobetaine, sulfobetaine, hydroxysulfobetaine, and amidosulfobetaine.

<8> The hair cleansing composition according to any one of <1> to <7>, wherein the anionic surfactant in the component (b) comprises preferably one or more selected from the group consisting of an N-acylamino acid salt, an alkyl ether carboxylate, an α-olefin sulfonate, a polyoxyethylene alkyl ether sulfate, and an alkyl ether sulfate, more preferably one or more selected from the group consisting of an alkyl ether carboxylate, an α-olefin sulfonate, a polyoxyethylene alkyl ether sulfate, and an alkyl ether sulfate, further more preferably one or more selected from the group consisting of an α-olefin sulfonate and a polyoxyethylene alkyl ether sulfate, and further more preferably a polyoxyethylene alkyl ether sulfate.

<9> The hair cleansing composition according to any one of <1> to <8>, wherein the amphoteric surfactant in the component (b) is preferably one or more selected from the group consisting of carbobetaine and sulfobetaine, more preferably one or more selected from the group consisting of a fatty acid amidoalkyl betaine and an alkyl hydroxysulfobetaine, further more preferably one or more selected from the group consisting of a fatty acid amidopropyl betaine having a fatty acid group having from 8 to 14 carbon atoms and an alkyl hydroxysulfobetaine having an alkyl group having from 8 to 14 carbon atoms, and further more preferably one or more selected from the group consisting of lauramidopropyl betaine and lauryl hydroxysultaine.

<10> The hair cleansing composition according to any one of <1> to <9>, wherein the component (b) comprises preferably one or more anionic surfactants selected from the group consisting of a carboxylic acid-type anionic surfactant, a sulfonic acid-type anionic surfactant, and an ether sulfate-type anionic surfactant, and one or more amphoteric surfactants.

<11> The hair cleansing composition according to any one of <1> to <10>, wherein a content of the component (b) is 5% by mass or more, preferably 8% by mass or more, and more preferably 12% by mass or more, and is preferably 30% by mass or less, more preferably 25% by mass or less, and further more preferably 20% by mass or less.

<12> The hair cleansing composition according to any one of <1> to <11>, wherein the component (c) is preferably an organic acid having 8 or less of carbon atoms or a salt thereof, more preferably a dicarboxylic acid, a tricarboxylic acid, a hydroxycarboxylic acid, a carboxylic acid having an aldehyde group, each having 8 or less carbon atoms, or a salt thereof, and further more preferably a hydroxycarboxylic acid having 8 or less of carbon atoms, or a salt thereof.

<13> The hair cleansing composition according to any one of <1> to <12>, wherein the component (c) is preferably one or more hydroxycarboxylic acids selected from the group consisting of glycolic acid, lactic acid, malic acid, tartaric acid, and citric acid, or a salt thereof, more preferably one or more hydroxycarboxylic acids selected from the group consisting of lactic acid, malic acid, and tartaric acid, or a salt thereof, and further more preferably one or more hydroxycarboxylic acids selected from the group consisting of lactic acid and malic acid, or a salt thereof.

<14> The hair cleansing composition according to any one of <1> to <13>, wherein a content of the component (c) is preferably 0.1% by mass or more, more preferably 0.2% by mass or more, and further more preferably 0.5% by mass or more, and is preferably 5% by mass or less, more preferably 2% by mass or less, and further more preferably 1% by mass or less.

<15> The hair cleansing composition according to any one of <1> to <14>, wherein a mass ratio of the component (a) to the component (c), (a)/(c), is preferably 0.5 or more, more preferably 1.0 or more, further more preferably 3 or more, and further more preferably 5 or more, and is preferably 100 or less, more preferably 50 or less, further more preferably 30 or less, and further more preferably 25 or less.

<16> The hair cleansing composition according to any one of <1> to <15>, wherein the hair cleansing composition further comprises one or more nonionic surfactants as component (d).

<17> The hair cleansing composition according to <16>, wherein the component (d) is preferably one or more selected from the group consisting of an alkyl glucoside, a sorbitan fatty acid ester, a glycerin fatty acid ester, a polyglycerin fatty acid ester, a propylene glycol fatty acid ester, a polyethylene glycol fatty acid ester, a sucrose fatty acid ester, a polyoxyethylene fatty acid ester, a polyoxyethylene sorbitan fatty acid ester, a polyoxyethylene alkyl ether, a polyoxypropylene alkyl ether, an alkyl glyceryl ether, a polyoxyethylene sorbitol fatty acid ester, a polyoxyethylene glycerin fatty acid ester, a polyoxyethylene propylene glycol fatty acid ester, polyoxyethylene castor oil, polyoxyethylene hydrogenated castor oil, a polyoxyethylene hydrogenated castor oil fatty acid ester, polyoxyethylene phytostanol ether, polyoxyethylene phytosterose ether, polyoxyethylene cholestanol ether, polyoxyethylene cholesteryl ether, a polyoxyalkylene modified organopolysiloxane, and a polyoxyalkylene/alkyl co-modified organopolysiloxane, more preferably one or more selected from the group consisting of an alkyl glucoside, a polyoxyethylene alkyl ether, a polyoxypropylene alkyl ether, and an alkyl glyceryl ether, further more preferably one or more selected from the group consisting of an alkyl glucoside, a polyoxypropylene alkyl ether, and an alkyl glyceryl ether, and further more preferably an alkyl glucoside.

<18> The hair cleansing composition according to <16> or <17>, wherein a content of the component (d) is preferably 0.1% by mass or more, more preferably 0.5% by mass or more, and further more preferably 1% by mass or more, and is preferably 10% by mass or less, more preferably 8% by mass or less, and further more preferably 5% by

mass or less.
<19> The hair cleansing composition according to any one of <1> to <18>, wherein the hair cleansing composition preferably further comprises a cationic polymer as component (e).
<20> The hair cleansing composition according to <19>, wherein the component (e) comprises preferably one or more selected from the group consisting of cationized cellulose, cationized starch, cationized guar gum, cationized tara gum, cationized fenugreek gum, and cationized locust bean gum, more preferably one or more selected from the group consisting of cationized cellulose and cationized guar gum, and further more preferably cationized cellulose.
<21> The hair cleansing composition according to <19> or <20>, wherein a content of the component (e) is preferably 0.01% by mass or more, more preferably 0.1% by mass or more, and further more preferably 0.2% by mass or more, and is preferably 5% by mass or less, more preferably 3% by mass or less, and further more preferably 1% by mass or less.
<22> The hair cleansing composition according to any one of <1> to <21>, wherein a content of the anionic or amphoteric film-forming polymer is preferably 5% by mass or less, more preferably 1% by mass or less, further more preferably 0.5% by mass or less, and even more preferably 0.1% by mass or less, and the hair cleansing composition is even more preferably substantially free of the anionic or amphoteric film-forming polymer.
<23> The hair cleansing composition according to any one of <1> to <22>, wherein the hair cleansing composition is substantially free of a reductive compound selected from the group consisting of a compound having a thiol group or an aldehyde group, a sulfite, and a sulfinic acid.
<24> The hair cleansing composition according to any one of <1> to <23>, wherein a content of a reductive compound having an ascorbyl group is preferably less than 1% by mass, more preferably less than 0.5% by mass, further more preferably less than 0.1% by mass, and even more preferably less than 0.05% by mass.
<25> The hair cleansing composition according to any one of <1> to <24>, wherein a pH at 25°C when the hair cleansing composition is diluted 20-fold by mass with water is preferably 3 or more, more preferably 3.5 or more, further more preferably 4 or more, and further more preferably 5 or more, and is preferably 8 or less, more preferably 7.5 or less, further more preferably 7 or less, and further more preferably 6 or less.
<26> A method for treating hair, comprising applying the hair cleansing composition according to any one of <1> to <25> to hair having waves or curls well moistened with water, letting the same sit on the entire hair, well lathering the same, and then washing off the same, and then carrying out a scrunch operation while the hair is still wet.
<27> The method for treating hair according to <26>, wherein the scrunch operation is preferably one or more operations selected from the group consisting of the following 1) to 3) :

1) the hair is grasped or massaged
2) the hair is twisted
3) the hair is placed in a palm of a hand, and then lifted up in such a way as to grasp up the hair and released.

<28> A hair cleansing composition comprising the following components (a), (b), and (c):

(a) one or more compounds selected from the group consisting of diethylene glycol monoethyl ether (ethyl carbitol), diethylene glycol monobutyl ether (butyl carbitol), triethylene glycol monomethyl ether, and 3-methyl-1,3-butanediol (isoprene glycol) at 0.1% by mass or more and 18% by mass or less;
(b) one or more selected from the group consisting of an N-acylamino acid salt, an alkyl ether carboxylate, an $\alpha$-olefin sulfonate, a polyoxyethylene alkyl ether sulfate, an alkyl ether sulfate, a fatty acid amidoalkyl betaine, and an alkyl hydroxysulfobetaine at 5% by mass or more and 30% by mass or less; and
(c) one or more hydroxycarboxylic acids selected from the group consisting of glycolic acid, lactic acid, malic acid, tartaric acid, and citric acid, or a salt thereof at 0.1% by mass or more and 5% by mass or less.

<29> A hair cleansing composition comprising the following components (a), (b), and (c):

(a) one or more compounds selected from the group consisting of diethylene glycol monoethyl ether (ethyl carbitol), diethylene glycol monobutyl ether (butyl carbitol), and triethylene glycol monomethyl ether at 2.0% by mass or more and 12% by mass or less;
(b) one or more selected from the group consisting of an N-acylamino acid salt, an alkyl ether carboxylate, an $\alpha$-olefin sulfonate, and a polyoxyethylene alkyl ether sulfate, and an alkyl hydroxysulfobetaine at 8% by mass or more and 25% by mass or less; and
(c) one or more hydroxycarboxylic acids selected from the group consisting of glycolic acid, lactic acid, malic acid, tartaric acid, and citric acid, or a salt thereof at 0.2% by mass or more and 2% by mass or less.

<30> A hair cleansing composition comprising the following components (a), (b), (c), and (d):

(a) one or more compounds selected from the group consisting of diethylene glycol monoethyl ether (ethyl carbitol), diethylene glycol monobutyl ether (butyl carbitol), and triethylene glycol monomethyl ether at 2.0% by mass or more and 12% by mass or less;
(b) one or more selected from the group consisting of an N-acylamino acid salt, an alkyl ether carboxylate, an α-olefin sulfonate, and a polyoxyethylene alkyl ether sulfate, and an alkyl hydroxysulfobetaine at 8% by mass or more and 25% by mass or less;
(c) one or more hydroxycarboxylic acids selected from the group consisting of glycolic acid, lactic acid, malic acid, tartaric acid, and citric acid, or a salt thereof at 0.2% by mass or more and 2% by mass or less; and
(d) one or more selected from the group consisting of an alkyl glucoside, a polyoxyethylene alkyl ether, a polyoxypropylene alkyl ether, and an alkyl glyceryl ether at 0.5% by mass or more and 8% by mass or less.

<31> A hair cleansing composition comprising the following components (a), (b), (c), and (e):

(a) one or more compounds selected from the group consisting of diethylene glycol monoethyl ether (ethyl carbitol), diethylene glycol monobutyl ether (butyl carbitol), and triethylene glycol monomethyl ether at 2.0% by mass or more and 12% by mass or less;
(b) one or more selected from the group consisting of an N-acylamino acid salt, an alkyl ether carboxylate, an α-olefin sulfonate, and a polyoxyethylene alkyl ether sulfate, and an alkyl hydroxysulfobetaine at 8% by mass or more and 25% by mass or less;
(c) one or more hydroxycarboxylic acids selected from the group consisting of glycolic acid, lactic acid, malic acid, tartaric acid, and citric acid, or a salt thereof at 0.2% by mass or more and 2% by mass or less; and
(e) one or more selected from the group consisting of cationized cellulose and cationized guar gum at 0.1% by mass or more and 3% by mass or less.

<32> A hair cleansing composition comprising the following components (a), (b), (c), (d), and (e):

(a) one or more compounds selected from the group consisting of diethylene glycol monoethyl ether (ethyl carbitol), diethylene glycol monobutyl ether (butyl carbitol), and triethylene glycol monomethyl ether at 2.0% by mass or more and 12% by mass or less;
(b) one or more selected from the group consisting of an N-acylamino acid salt, an alkyl ether carboxylate, an α-olefin sulfonate, and a polyoxyethylene alkyl ether sulfate, and an alkyl hydroxysulfobetaine at 8% by mass or more and 25% by mass or less;
(c) one or more hydroxycarboxylic acids selected from the group consisting of glycolic acid, lactic acid, malic acid, tartaric acid, and citric acid, or a salt thereof at 0.2% by mass or more and 2% by mass or less;
(d) one or more selected from the group consisting of an alkyl glucoside, a polyoxyethylene alkyl ether, a polyoxypropylene alkyl ether, and an alkyl glyceryl ether at 0.5% by mass or more and 8% by mass or less; and
(e) one or more selected from the group consisting of cationized cellulose and cationized guar gum at 0.1% by mass or more and 3% by mass or less.

<33> The hair cleansing composition according to any one of <28> to <32>, wherein a mass ratio of the component

(a) to the component (c), (a)/(c), is 0.5 or more and 100 or less.

<34> The hair cleansing composition according to any one of <28> to <32>, wherein a mass ratio of the component

(a) to the component (c), (a)/(c), is 1.0 or more and 50 or less.

<35> The hair cleansing composition according to any one of <28> to <32>, wherein a mass ratio of the component

(a) to the component (c), (a)/(c), is 3 or more and 30 or less.

<36> The hair cleansing composition according to any one of <28> to <32>, wherein a mass ratio of the component (a) to the component (c), (a)/(c), is 5 or more and 25 or less.

Examples

Examples 1 to 18 and Comparative Examples 1 to 6

Shampoos shown in Tables 3 to 5 were prepared and evaluated as follows. In addition, the following plain shampoo and conditioner were used for hair treatment.

[Table 1]

| Plain shampoo formulation | | |
|---|---|---|
| Raw material name | Component name | Content (% by mass) |
| Purified water | | Balance |
| EMAL 227-pH11 (manufactured by Kao Corporation) | Na laureth (2 mol) sulfate | 15.498 |
| Clewat N (manufactured by Nagase ChemteX Corporation) | Disodium edetate | 0.15 |
| Sodium benzoate solution (35%) | | 0.175 |
| AMINON L-02 (manufactured by Kao Corporation) | Lauramide DEA | 2.28 |
| Phosphoric acid (85%) | | 0.068 |
| NaCl | | 0.8 |
| Fragrance | | 0.4 |

[Table 2]

| Conditioner formulation | | |
|---|---|---|
| Raw material name | Component name | Content (% by mass) |
| Purified water | | 88 |
| KALCOL 6870 (manufactured by Kao Corporation) | Cetanol | 5 |
| QUARTAMIN 86W (manufactured by | Stearyltrimethylammo | 7 |
| Kao Corporation) | nium chloride | |
| Fermented lactic acid | Lactic acid | q.s. |
| | pH | 3.5 |

(Tresses for evaluation)

0.25 g of Caucasian curly hair (purchased from Kerling International Haarrfabrik, 28 cm long when it is stretched, one having 4 to 5 curls from the root to the hair end picked) was used as one tress and treated as follows.

Untreated (control):

The plain shampoo was applied to the tresses (0.1 g applied to 0.25 g of tresses; bath ratio of 1 : 0.4), worked into lather to wash the tresses for 1 minute, and then rinsed off with running water for 30 seconds. After it was rinsed, excess moisture was removed from the hair, then the conditioner was applied (0.1 g applied to 0.25 g of tresses; bath ratio of 1:0.4), allowed to stand for 1 minute, and then rinsed off with running water for 30 seconds.

Examples and Comparative Examples:

The shampoo described in each of the Examples and the Comparative Examples was applied to the tresses (0.1 g applied to 0.25 g of tresses; bath ratio of 1 : 0.4), worked into lather to wash the tresses for 1 minute, and then rinsed off with running water for 30 seconds. After it was rinsed, excess moisture was removed from the hair, then the conditioner was applied (0.1 g applied to 0.25 g of tresses; bath ratio of 1:0.4), allowed to stand for 1 minute, and then rinsed off with running water for 30 seconds.

(Evaluation procedures)

The following evaluations were carried out on the curly hair tresses after hair treatment.

• Spiral rate (%)

The tresses after rinsing was allowed to drain naturally without moving the same for 10 seconds, and then subjected to scrunch treatment 10 times on a towel.

As exemplified in Figure 1, the tresses are placed from the hair end in such a way as to fold the tresses, a curl is counted every half turn of the curl, and when the direction of the curl is observed from the root side of the tresses, a point where a certain curl curls in the same direction as half a turn before the curl (forward curve point) is considered to be a beautiful curl created there. A larger number of forward curve points indicates that the curls are more consistent. According to the following equation, the ratio of the total number of forward curve points to the total number of curl points of the tresses is defined as the spiral rate. In the evaluation, the average value of the spiral rate of 5 tresses was adopted.

Spiral rate (%) = (number of forward curve points/total number of curl points) × 100

wherein the "number of forward curve points" is the number of points where a certain curl curls in the same direction as half a turn before the curl when tresses are placed from the hair end in such a way as to fold the tresses and the direction of the curl is observed from the root side of the tresses.

For example, in the example on the left side of Figure 1, all four curls counted every half turn of the curl are forward curve points, and thus the spiral rate is (4/4) × 100 = 100%, and in the example on the right side of Figure 1, three of the four curls are forward curve points and one is a backward curve point, and thus the spiral rate is (3/4) × 100 = 75%. It is determined that the higher the spiral rate, the more uniform the curl.

• Manageability of curl

After rinsing, the tresses were towel-dried, and dried for 3 minutes by using a diffuser. The tresses after drying was fixed at one end thereof and hung, and then photographed from the front. One professional evaluator determined the manageability of the tresses on a 5-point scale from the photograph of the tresses.

Comparative Example 1 was given a score of 5 for tresses having a wide tress width (loose), Example 1 was given a score of 1 for tresses having a narrow width (manageable), the difference between the tress widths of score 1 and score 5 was divided into 5 equal parts to create other scores, and the evaluation was carried out by using these scores.

• Uniformity of curl

After rinsing, the tresses were towel-dried, and dried for 3 minutes by using a diffuser. The tresses after drying was fixed at one end thereof and hung, and then photographed from the front. The curl radius of the tresses was measured from the photograph of the tresses, and the standard deviation thereof was calculated from the following equation.

It is determined that the smaller the standard deviation, the more uniform the curl.

$$s = \sqrt{\frac{1}{n}\sum_{i=1}^{n}(x_i - \bar{x})^2}$$

s : standard deviation
n : total number of data
$x_i$ :value of each data
$\bar{x}$ : average of data

• Measurement of pH

The shampoo described in each of the Examples and the Comparative Examples was diluted 20-fold by mass with water and stirred well, and then the pH thereof was measured at 25°C by using a pH meter (HM-30R, manufactured by DKK-TOA Corporation).

[Table 3]

| Components (% by mass; all active amounts) | | Examples | | | | | | | Comparative Examples | | |
|---|---|---|---|---|---|---|---|---|---|---|---|
| | | 1 | 2 | 3 | 4 | 5 | 6 | 7 | 1 | 2 | 3 |
| (a) | Diethylene glycol monoethyl ether | 10 | - | - | - | 1 | 2 | 15 | - | - | 20 |
| | Diethylene glycol monobutyl ether | - | 10 | - | - | - | - | - | - | - | - |
| | Triethylene glycol monomethyl ether | - | - | 10 | - | - | - | - | - | - | - |
| | Isoprene glycol | - | - | - | 10 | - | - | - | - | - | - |
| (a') | Dipropylene glycol | - | - | - | - | - | - | - | - | 10 | - |
| (b) | Polyoxyethylene lauryl ether ammonium sulfate (*1) | 12 | 12 | 12 | 12 | 12 | 12 | 12 | 12 | 12 | 12 |
| | Lauryl hydroxysultaine (*2) | 3 | 3 | 3 | 3 | 3 | 3 | 3 | 3 | 3 | 3 |
| (c) | Lactic acid | 0.5 | 0.5 | 0.5 | 0.5 | 0.5 | 0.5 | 0.5 | 0.5 | 0.5 | 0.5 |
| (d) | Decyl glucoside (*3) | 1 | 1 | 1 | 1 | 1 | 1 | 1 | 1 | 1 | 1 |
| (e) | Polyquaternium-10 (*4) | 0.5 | 0.5 | 0.5 | 0.5 | 0.5 | 0.5 | 0.5 | 0.5 | 0.5 | 0.5 |
| Evaluations | Spiral rate (%) | 64 | 64 | 65 | 56 | 52 | 60 | 49 | 39 | 34 | 41 |
| | Curl manageability (score) | 1 | 1 | 2 | 3 | 3 | 3 | 3 | 5 | 4 | 4 |
| | Curl uniformity (standard deviation of curl radius) | 0.28 | 0.35 | 0.38 | 0.75 | 0.67 | 0.44 | 0.55 | 1.96 | 1.07 | 1.12 |

*1: EMAL 125A (manufactured by Kao Corporation)
*2: AMPHITOL 20HD*MIX (manufactured by Kao Corporation)
*3: MYDOL 10 (manufactured by Kao Corporation)
*4: CATICELLO L-150 (manufactured by Kao Corporation)

[Table 4]

| Components (% by mass; all active amounts) | | Examples | | | | | | | Comparative Examples | |
|---|---|---|---|---|---|---|---|---|---|---|
| | | 8 | 9 | 10 | 11 | 12 | 13 | 14 | 4 | 5 |
| (a) | Diethylene glycol monoethyl ether | 10 | 10 | 10 | 10 | 10 | 10 | 10 | 10 | 10 |

(continued)

| Components (% by mass; all active amounts) | | Examples | | | | | | | Comparative Examples | |
|---|---|---|---|---|---|---|---|---|---|---|
| | | 8 | 9 | 10 | 11 | 12 | 13 | 14 | 4 | 5 |
| (b) | Polyoxyethylene lauryl ether ammonium sulfate (*1) | 5 | 18 | 22 | - | - | - | 15 | 0.5 | 2 |
| | Na cocoyl glutamate (*5) | - | - | - | 12 | - | - | - | - | - |
| | Na laureth-3 carboxylate (*6) | - | - | - | - | 12 | - | - | - | - |
| | Sodium tetradecene sulfonate (*7) | - | - | - | - | - | 12 | - | - | - |
| | Lauryl hydroxysultaine (*2) | - | 3 | 3 | 3 | 3 | 3 | - | - | - |
| (c) | Lactic acid | 0.5 | 0.5 | 0.5 | 0.5 | 0.5 | 0.5 | 0.5 | 0.5 | 0.5 |
| (d) | Decyl glucoside (*3) | 1 | 1 | 1 | 1 | 1 | 1 | 1 | 1 | 1 |
| (e) | Polyquaternium-10 (*4) | 0.5 | 0.5 | 0.5 | 0.5 | 0.5 | 0.5 | 0.5 | 0.5 | 0.5 |
| Evaluations | Spiral rate (%) | 50 | 51.6 | 51 | 50 | 52 | 56.6 | 56 | 37 | 42 |
| | Curl manageability (score) | 3 | 3 | 2 | 2 | 2 | 2 | 2 | 4 | 4 |
| | Curl uniformity (standard deviation of curl radius) | 0.56 | 0.23 | 0.45 | 0.48 | 0.68 | 0.20 | 0.23 | 1.05 | 1.09 |

*1: EMAL 125A (manufactured by Kao Corporation)
*2: AMPHITOL 20HD*MIX (manufactured by Kao Corporation)
*3: MYDOL 10 (manufactured by Kao Corporation)
*4: CATICELLO L-150 (manufactured by Kao Corporation)
*5: AMISOFT CS-22B (manufactured by Ajinomoto Healthy Supply Co., Inc.)
*6: AKYPO LM-26SD (manufactured by Kao Corporation)
*7: K LIPOLAN PJ-400C (manufactured by Lion Specialty Chemicals Co., Ltd.)

[Table 5]

| Components (% by mass; all active amounts) | | Examples | | | | Comparative Examples |
|---|---|---|---|---|---|---|
| | | 15 | 16 | 17 | 18 | 6 |
| (a) | Diethylene glycol monoethyl ether | 10 | 10 | 10 | 10 | 10 |
| (b) | Polyoxyethylene lauryl ether ammonium sulfate (*1) | 12 | 12 | 12 | 12 | 12 |
| | Lauryl hydroxysultaine (*2) | 3 | 3 | 3 | 3 | 3 |
| (c) | Lactic acid | 0.5 | 0.5 | 0.5 | 0.5 | - |

(continued)

| Components (% by mass; all active amounts) | | Examples | | | | Comparative Examples |
|---|---|---|---|---|---|---|
| | | 15 | 16 | 17 | 18 | 6 |
| (d) | Decyl glucoside (*3) | - | - | - | 1 | 1 |
| | Isodecyl glyceryl ether (*8) | 1 | - | - | - | - |
| | PPG-3 caprylyl ether (*9) | - | 1 | - | - | - |
| (e) | Polyquaternium-10 (*4) | 0.5 | 0.5 | 0.5 | - | 0.5 |
| Evaluations | Spiral rate (%) | 58 | 52 | 47 | 64 | 41 |
| | Curl manageability (score) | 2 | 3 | 2 | 1 | 5 |
| | Curl uniformity (standard deviation of curl radius) | 0.30 | 0.58 | 0.32 | 0.33 | 1.17 |
| *1: EMAL 125A (manufactured by Kao Corporation)<br>*2: AMPHITOL 20HD*MIX (manufactured by Kao Corporation)<br>*3: MYDOL 10 (manufactured by Kao Corporation)<br>*4: CATICELLO M-80 (manufactured by Kao Corporation)<br>*8: PENETOL GE-ID (manufactured by Kao Corporation)<br>*9: KAO SOFCARE GP-1 (manufactured by Kao Corporation) | | | | | | |

When the shampoos used in the Examples and the Comparative Examples are diluted 20-fold by mass with water, the pH thereof at 25°C is in the range of from 3.5 to 7.5.

From Tables 3 to 5, it can be seen that the hair cleansing compositions of the present Examples can make the wet hair after hair washing more likely to form a spiral shape and can form a uniformly curl-consistent, three-dimensional, manageable, and beautiful waves or curls.

Formulation examples 1 to 3

Table 6 shows formulation examples of the hair cleansing composition of the present invention.

[Table 6]

| Components (% by mass; all active amounts) | | Formulation examples | | |
|---|---|---|---|---|
| | | 1 | 2 | 3 |
| (a) | Diethylene glycol monoethyl ether | 10 | 10 | 10 |
| (b) | Sodium polyoxyethylene lauryl ether sulfate (EMAL 227, manufactured by Kao Corporation) | - | 10 | - |
| | Ammonium polyoxyethylene lauryl ether sulfate (EMAL 125A, manufactured by Kao Corporation) | 13.5 | - | 11 |
| | Na cocoyl glutamate (AMISOFT CS-22B, manufactured by Kao Corporation) | - | 1.4 | - |
| | Na laureth-4 carboxylate (AKYPO LM-26SD manufactured by Kao Corporation) | - | - | 0.7 |
| | Lauryl hydroxysultaine (AMPHITOL 20HD*MIX, manufactured by Kao Corporation) | 2.2 | - | 0.8 |
| | Lauromidopropyl betaine (AMPHITOL 20AB, manufactured by Kao Corporation) | 1.2 | 2.9 | 0.6 |

(continued)

| Components (% by mass; all active amounts) | | Formulation examples 1 | 2 | 3 |
|---|---|---|---|---|
| (c) | Glyoxylic acid | - | 0.05 | - |
| | Lactic acid | - | 0.9 | - |
| | Malic acid | 1.5 | - | 0.33 |
| | 2Na succinate | - | - | 0.14 |
| (d) | Polyoxyethylene lauryl ether (3EO) (EMULGEN 103KG, manufactured by Kao Corporation) | 0.2 | - | - |
| | Polyoxyethylene lauryl ether (16EO) (EMULGEN 116, manufactured by Kao Corporation) | 2 | - | - |
| | Isodecyl glyceryl ether (PENETOL GE-ID, manufactured by Kao Corporation) | 0.95 | - | 0.5 |
| | PPG-3 caprylyl ether (KAO SOFCARE GP-1, manufactured by Kao Corporation) | 0.75 | - | 0.6 |
| (e) | Polyquaternium-10 (CATICELLO L-150, manufactured by Kao Corporation) | 0.04 | - | 0.2 |
| | Polyquaternium-10 (CATICELLO M-80, manufactured by Kao Corporation) | 0.31 | - | - |
| | Guar Hydroxypropyltrimonium chloride (Jaguar C-14SK (US)(CN), manufactured by Solvay) | - | - | 0.32 |

**Claims**

**1.** A hair cleansing composition comprising the following components (a), (b), and (c):

(a) one or more compounds selected from the group consisting of a polyalkylene glycol ether and an alkylene glycol at 0.1% by mass or more and 18% by mass or less;
(b) one or more selected from the group consisting of a carboxylic acid-type anionic surfactant, a sulfonic acid-type anionic surfactant, an ether sulfate-type anionic surfactant, and an amphoteric surfactant at 5% by mass or more; and
(c) an organic acid or a salt thereof.

**2.** The hair cleansing composition according to claim 1, wherein a content of the component (a) is 0.5% by mass or more and 15% by mass or less.

**3.** The hair cleansing composition according to claim 1 or 2, wherein a content of the component (b) is 8% by mass or more and 30% by mass or less.

**4.** The hair cleansing composition according to any one of claims 1 to 3, wherein the component (c) is a hydroxycarboxylic acid.

**5.** The hair cleansing composition according to any one of claims 1 to 4, wherein the component (b) comprises one or more anionic surfactants selected from the group consisting of a carboxylic acid-type anionic surfactant, a sulfonic acid-type anionic surfactant, and an ether sulfate-type anionic surfactant, and one or more amphoteric surfactants.

**6.** The hair cleansing composition according to any one of claims 1 to 5, wherein the hair cleansing composition further

comprises one or more nonionic surfactants as component (d).

**7.** The hair cleansing composition according to any one of claims 1 to 6, wherein a mass ratio of the component (a) to the component (c), (a)/(c), is 0.5 or more and 100 or less.

Fig. 1

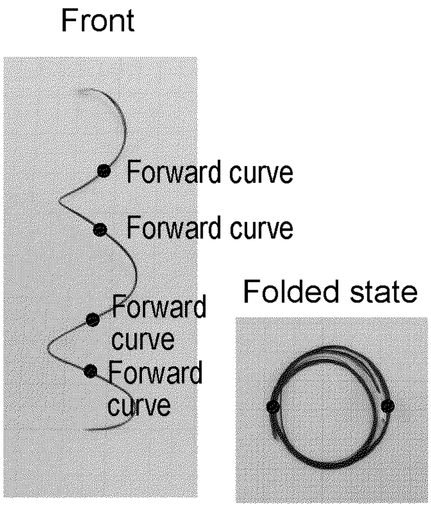


$$\frac{4}{4} \times 100 = 100\ \%$$

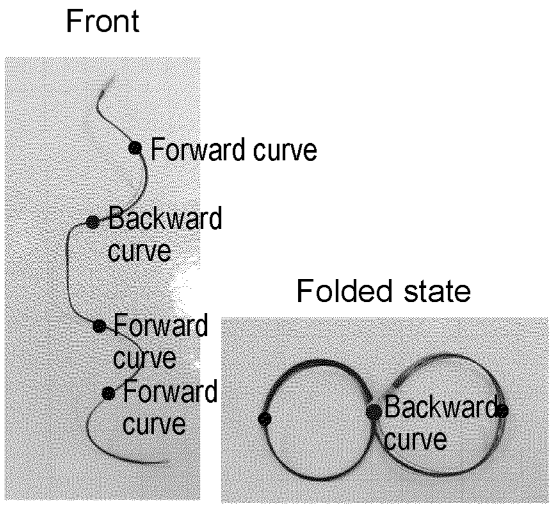


$$\frac{3}{4} \times 100 = 75\ \%$$

**INTERNATIONAL SEARCH REPORT**

International application No.
**PCT/JP2022/044607**

**A. CLASSIFICATION OF SUBJECT MATTER**

***A61Q 5/02***(2006.01)i; ***A61Q 5/12***(2006.01)i; ***A61K 8/34***(2006.01)i; ***A61K 8/36***(2006.01)i; ***A61K 8/39***(2006.01)i; ***A61K 8/46***(2006.01)i; ***A61K 8/86***(2006.01)i
FI: A61K8/36; A61Q5/02; A61K8/86; A61K8/39; A61K8/34; A61K8/46; A61Q5/12

According to International Patent Classification (IPC) or to both national classification and IPC

**B. FIELDS SEARCHED**

Minimum documentation searched (classification system followed by classification symbols)

A61Q1/00-90/00; A61K8/00-8/99

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2012-207019 A (SHISEIDO CO LTD) 25 October 2012 (2012-10-25) paragraphs [0078], [0095] | 1-7 |
| Y | | 1-7 |
| X | JP 2020-512979 A (L'OREAL) 30 April 2020 (2020-04-30) paragraphs [0001], [0010], [0074], [0253] | 1-7 |
| X | JP 4-230614 A (KAO CORP) 19 August 1992 (1992-08-19) paragraphs [0049]-[0051] | 1-3, 5-7 |
| Y | | 4 |
| Y | JP 2003-212733 A (KAO CORP) 30 July 2003 (2003-07-30) paragraphs [0028], [0032] | 1-7 |

☐ Further documents are listed in the continuation of Box C.   ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 January 2023** | **07 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/044607**

| Patent document cited in search report | Publication date (day/month/year) | Patent family member(s) | Publication date (day/month/year) |
|---|---|---|---|
| JP 2012-207019 A | 25 October 2012 | (Family: none) | |
| JP 2020-512979 A | 30 April 2020 | US 2018/0280270 A1<br>paragraphs [0002], [0015], [0088]-[0095], [0509]<br>WO 2018/183858 A1<br>EP 3615040 A1<br>CN 110461341 A | |
| JP 4-230614 A | 19 August 1992 | (Family: none) | |
| JP 2003-212733 A | 30 July 2003 | US 2003/0170197 A1<br>paragraphs [0034], [0038]<br>EP 1329214 A2<br>CN 1433749 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4230614 A
- JP 2015105271 A
- JP 2012131784 A
- JP 2020186222 A
- JP H1171435 A
- JP S53139734 A
- JP S6036407 A